# EUROPEAN PATENT SPECIFICATION

(11) **EP 2 876 098 B1**
(45) Date of publication and mention of the grant of the patent: **13.07.2016**
(21) Application number: 13306592.0
(22) Date of filing: 21.11.2013
(51) Int. Cl.: C07C 7/00, C07C 7/08, C07C 7/20, C07C 13/39

(54) **Extractive distillation in a norbornadiene purification method**
Reinigung von Norbornadien durch Extraktivdestillation
Purification du norbornadiene par distillation extractive

(43) Date of publication of application: 27.05.2015
(73) Proprietor: L'AIR LIQUIDE, SOCIETE ANONYME POUR L'ETUDE ET L'EXPLOITATION DES PROCEDES GEORGES CLAUDE, 75007 Paris (FR)
(72) Inventor: Danvel, Laurent, 21630 POMMARD (FR); Plaza, Sonia, 71100 CHALON SUR SAONE (FR)
(74) Representative: Grout de Beaufort, François-Xavier

(56) References cited:
- US-A- 5 458 741
- US-A1- 2009 159 843

## Description

The present invention concerns a new method for improving BCHD (Norbornadiene, BisCycloHeptaDiene) purity by removing the benzene usually present. Benzene being a carcinogenic material, the purified compounds has a reduced toxicity.

Purified norbornadiene can then be used to in vapor deposition methods such as (plasma-enhanced) chemical vapor deposition for semiconductor manufacturing.

In semiconductor and electronics industries, device shrinking allows higher performances and processing speed.

However reducing the device dimensions led to an increase of defects like leakage current through the insulators or increase capacitance in BEOL leading to slower chips speed.

As components have shrinked and transistors are closer, the insulating dielectrics (low-k) have thinned to the point where charge build up and crosstalk negatively affect the device performances. Replacing the SiO₂ or SiO₂:F with a low dielectric constant (k) that is know a porous SiOC film of the same thickness reduces parasitic capacitance, enabling faster switching speeds and lower heat dissipation.

Those porous low-k films are traditionally deposited using SiCₓO_{y}H_{z} containing precursors such as for instance, DEOMS, DEMS, 4MS, 3MS, OMCTS, TMCTS in combination with a porogen such as ATPR, BCHD or CₓH_{z} where x, y and z are integers.

Additional gases such as O₂, N₂, N₂O, NO₂, NO, Ar or other inert gases can be introduced to tune the process. The porogen will be incorporated into the film and after deposition the film may be cured (exposed to UV or a remote plasma) for less than 10 minutes.

During the curing time, the porogen is removed from the films leaving voids called pores that allow a decrease of the dielectric constant of the film (k or air = 1). Examples of UV curing processes can be found in US6756085.

BCHD and benzene have very close boiling point and can not be removed by standard distillation (Benzene BP=80,1°C - BCHD BP=89°C).

Benzene presents however serious safety risks causing cancer or other illness. It is also an impurity in the BCHD that is preferred to be used in ultrahigh purity in microelectronics to enhance yield of processes and limit process variations.

US 2009/159843 discloses a method for the preparation of a composition comprising BCHD comprising the step of flash distillation to remove the non-volatile impurities.

By the term benzene-free BCHD the applicant means BCHD that containing residual amount of benzene, possibly undetected. It means benzene content below 10ppm, and more preferably below 1 ppm.

The present invention provides a method of removing benzene from raw BCHD. This allows a production of a benzene free BCHD that is safer to handle and could enhance yield control.

The object of the present invention is a method of preparation of a porogen composition comprising for 100% by weight:
- at least 99,5% by weight of norbornadiene ;
- from 10 ppm to 500 ppm by weight of a nitroxyl radical based ;
- from 250 ppb to 10 ppm by weight of benzene,
said method comprising:
step a) providing a benzene containing norbornadiene solution comprising more than 100 ppm of benzene,
step b) separating the benzene from norbornadiene by extractive distillation,
step c) adding to the norbornadiene solution issued from step b) a nitroxyl radical based stabilizer.

According to other embodiments, the present invention also concerns:

A method as defined above wherein the prepared porogen composition comprises less than 1 ppm of benzene.

A method as defined above wherein the prepared porogen composition comprises at least 99,95% of norbornadiene.

A method as defined above wherein the nitroxyl radical based stabilizer is selected from the group consisting of 2,2,6,6-tetramethyl-1-piperidinyloxy (TEMPO), 4-hydroxy-2,2,6,6-tetramethyl-1-piperidinyloxy (4H-TEMPO), di-tert-butyl nitroxyl, 1-oxyl-2,2,6,6-tetramethylpiperidin-4-one, 1-oxyl-2,2,6,6-tetramethylpiperidin-4-yl acetate, 1-oxyl-2,2,6,6-tetramethylpiperidin-4-yl 2-ethylhexanoate, 1-oxyl-2,2,6,6-tetramethylpiperidin-4-yl stearate, 1-oxyl-2,2,6,6-tetramethylpiperidin-4-yl benzoate, 1-oxyl-2,2,6,6-tetramethylpiperidin-4-yl 4-tert-butylbenzoate, bis(1-oxyl-2,2,6,6-tetramethylpiperidin-4-yl)succinate, bis(1-oxyl-2,2,6,6-tetramethylpiperidin-4-yl)adipate, bis(1-oxyl-2,2,6,6-tetramethylpiperidin-4-yl)sebacate, bis(1-oxyl-2,2,6,6-tetramethylpiperidin-4-yl)n-butylmalonate, bis(1-oxyl-2,2,6,6-tetramethylpiperidin-4-yl)phthalate, bis(1-oxyl-2,2,6,6-tetramethylpiperidin-4-yl)isophthalate, bis(1-oxyl-2,2,6,6-tetramethylpiperidin-4-yl)terephthalate, bis(1-oxyl-2,2,6,6-tetramethylpiperidin-4-yl)hexahydroter-ephthalate, N,N'-bis(1-oxyl-2,2,6,6-tetramethylpiperidin-4-yl)adipamide, N-(1-oxyl-2,2,6,6-tetramethylpiperidin-4-yl)-caprolactam, N-(1-oxyl-2,2,6,6-tetramethylpiperidin-4-yl)-dodecylsuccinimide, 2,4,6-tris(1-oxyl-2,2,6,6-tetrame-thylpiperidin-4-yl) isocyanurate, 2,4,6-tris-[N-butyl-N-1-oxyl-2,2,6,6-tetramethylpiperidin-4-yl]-s-triazine, 4,4'-ethyl-enebis(1-oxyl-2,2,6,6-tetramethylpiperazin-3-one) and combinations thereof.

A method as defined above, wherein the nitroxyl radical based stabilizer is selected from the group consisting of 2,2,6,6-tetramethyl-1-piperidinyloxy (TEMPO), 4-hydroxy-2,2,6,6-tetramethyl-1-piperidinyloxy (4H-TEMPO) and combinations thereof.

Extractive distillation is defined as distillation in the presence of a miscible, high boiling, relatively non-volatile component, the solvent, that forms no azeotrope with the other components in the mixture. The method is used for mixtures having a low value of relative volatility, nearing unity. Such mixtures cannot be separated by simple distillation, because the volatility of the two components in the mixture is nearly the same, causing them to evaporate at nearly the same temperature at a similar rate, making normal distillation impractical.

The method of extractive distillation uses a separation solvent, which is generally non-volatile, has a high boiling point and is miscible with the mixture, but doesn't form an azeotropic mixture. The solvent interacts differently with the components of the mixture thereby causing their relative volatilities to change. This enables the new three-part mixture to be separated by normal distillation. The original component with the greatest volatility separates out as the top product.

The bottom product consists of a mixture of the solvent and the other component, which can again be separated easily because the solvent does not form an azeotrope with it. The bottom product can be separated by any of the methods available.

It is important to select a suitable separation solvent for this type of distillation. The solvent must alter the relative volatility by a wide enough margin for a successful result. The quantity, cost and availability of the solvent should be considered. The solvent should be easily separable from the bottom product, and should not react chemically with the components or the mixture, or cause corrosion in the equipment.

In extractive distillation, an additional solvent, separating agent, with a high boiling point, is used to alter the relative volatility of the components to be separated.

In this way, it is possible to obtain one pure component at the top of the column and the other, together with the solvent at the bottom.

The distillation can be performed on a batch column.

If BCHD is retained in the boiler with the solvent, BCHD can be purified after the extractive distillation on the same column. The separation would be easy due to the high boiling point of the solvent.

If benzene is retained in the boiler with the solvent, according to the cost of the separating agent, one can choose to purify the solvent on the same column after the extractive distillation.

When performing extractive distillation between benzene and BCHD, the following solvents can be used: isoamyl formate, dimethylformamide, dimethylsulfoxide, ethyl acetoacetate, methyl acetoacetate, ethyl acetate, phenol, butyl phenol, 4-nitro phenol, 2-furaldehyde, isobutyl butyrate, cyanimide solvents.

Example of application of extractive distillation can be found in US 5 458 741. This patent studies the rise of relative volatility between benzene and cyclohexene using these extractive agents.

The effectiveness of these chemicals to perform extractive distillation between benzene and BCHD is not obvious.

Indeed:
- BCHD is a diene and cyclohexene contains only one double bond. Thus, between BCHD and cyclohexene, BCHD is functionally closer than benzene.
- US 5 458 741 studies the possibility to increase the relative volatility to obtain a final purity of 99% purifying a mixture containing 80% of benzene and 20% of cyclohexene. However, obtaining ultra-pure chemicals (purity > 99%) requires a number of theoretical plates more important.

Therefore, the extractive agents should increase significantly the relative volatility between BCHD and benzene in order to perform distillation with a number of theoretical plates between 10 and 40.

### Example

### Extractive distillation:

A batch distillation column is used. Thus, the solvent is introduced in the boiler with BCHD raw material. The solvent changes the relative volatility of benzene and BCHD and retains more likely benzene in the boiler.

The result of this distillation is a BCHD without benzene.

## Claims

1. A method of preparation of a porogen composition comprising for 100% by weight:
- at least 99,5% by weight of norbornadiene ;
- from 10 ppm to 500 ppm by weight of a nitroxyl radical based ;
- from 250 ppb to 10 ppm by weight of benzene,
said method comprising:
step a) providing a benzene containing norbornadiene solution comprising more than 100 ppm of benzene,
step b) separating the benzene from norbornadiene by extractive distillation,
step c) adding to the norbornadiene solution issued from step b) a nitroxyl radical based stabilizer.

2. Method according to Claim 1, wherein the porogen composition comprises at least 99,95% of norbornadiene.

3. Method according to one of preceding claims, wherein the nitroxyl radical based stabilizer is selected from the group consisting of 2,2,6,6-tetramethyl-1-piperidinyloxy (TEMPO), 4-hydroxy-2,2,6,6-tetramethyl-1-piperidinyloxy (4H-TEMPO), di-tert-butyl nitroxyl, 1-oxyl-2,2,6,6-tetramethylpiperidin-4-one, 1-oxyl-2,2,6,6-tetramethylpiperidin-4-yl acetate, 1-oxyl-2,2,6,6-tetramethylpiperidin-4-yl 2-ethylhexanoate, 1-oxyl-2,2,6,6-tetramethylpiperidin-4-yl stearate, 1-oxyl-2,2,6,6-tetramethylpiperidin-4-yl benzoate, 1-oxyl-2,2,6,6-tetramethylpiperidin-4-yl 4-tert-butylbenzoate, bis(1-oxyl-2,2,6,6-tetramethylpiperidin-4-yl)succinate, bis(1-oxyl-2,2,6,6-tetramethylpiperidin-4-yl)adipate, bis(1-oxyl-2,2,6,6-tetramethylpiperidin-4-yl)sebacate, bis(1-oxyl-2,2,6,6-tetramethylpiperidin-4-yl)n-butylmalonate, bis(1-oxyl-2,2,6,6-tetramethylpiperidin-4-yl)phthalate, bis(1-oxyl-2,2,6,6-tetramethylpiperidin-4-yl)isophthalate, bis(1-oxyl-2,2,6,6-tetramethylpiperidin-4-yl)terephthalate, bis(1-oxyl-2,2,6,6-tetramethylpiperidin-4-yl)hexahydroter-ephthalate, N,N'-bis(1-oxyl-2,2,6,6-tetramethylpiperidin-4-yl)adipamide, N-(1-oxyl-2,2,6,6-tetramethylpiperidin-4-yl)-caprolactam, N-(1-oxyl-2,2,6,6-tetramethylpiperidin-4-yl)-dodecylsuccinimide, 2,4,6-tris(1-oxyl-2,2,6,6-tetrame-thylpiperidin-4-yl) isocyanurate, 2,4,6-tris-[N-butyl-N-1-oxyl-2,2,6,6-tetramethylpiperidin-4-yl]-s-triazine, 4,4'-ethyl-enebis(1-oxyl-2,2,6,6-tetramethylpiperazin-3-one) and combinations thereof.

4. Method of Claim 3, wherein the nitroxyl radical based stabilizer is selected from the group consisting of 2,2,6,6-tetramethyl-1-piperidinyloxy (TEMPO), 4-hydroxy-2,2,6,6-tetramethyl-1-piperidinyloxy (4H-TEMPO) and combinations thereof.

5. Method according to anyone of preceding claims, wherein the porogen composition comprises less than 1 ppm of benzene.

## Patentansprüche

1. Verfahren zur Herstellung einer Porogenzusammensetzung, umfassend für 100 Gew.%:
- mindestens 99,5 Gew.% Norbornadien;
- von 10 Gew.ppm bis 500 Gew.ppm einer Nitroxylradikal-Basis;
- von 250 Gew.ppb bis 10 Gew.ppm Benzen,
wobei das Verfahren Folgendes umfasst:
Schritt a) Bereitstellen einer Benzen-enthaltenden Norbornadien-Lösung, umfassend mehr als 100 ppm Benzen,
Schritt b) Trennen des Benzens vom Norbornadien durch extraktive Destillation,
Schritt c) Zugeben zur Norbornadien-Lösung, die aus Schritt b) stammt, eines Nitroxylradikal-basierten Stabilisierungsmittels.

2. Verfahren nach Anspruch 1, wobei die Porogenzusammensetzung mindestens 99,95 % Norbornadien umfasst.

3. Verfahren nach einem der vorhergehenden Ansprüche, wobei das Nitroxylradikal-basierte Stabilisierungsmittel ausgewählt ist aus der Gruppe, bestehend aus 2,2,6,6-Tetramethyl-1-piperidinyloxy (TEMPO), 4-Hydroxy-2,2,6,6-tetramethyl-1-piperidinyloxy (4H-TEMPO), di-tert-Butylnitroxyl, 1-Oxyl-2,2,6,6-tetramethylpiperidin-4-on, 1-Oxyl-2,2,6,6-tetramethylpiperidin-4-ylacetat, 1-Oxyl-2,2,6,6-tetramethylpiperidin-4-yl 2-ethylhexanoat, 1-Oxyl-2,2,6,6-tetramethylpiperidin-4-ylstearat, 1-Oxyl-2,2,6,6-tetramethylpiperidin-4-ylbenzoat, 1-Oxyl-2,2,6,6-tetramethylpiperidin-4-yl 4-tert-butylbenzoat, bis(1-Oxyl-2,2,6,6-tetramethylpiperidin-4-yl)succinat, bis(1-Oxyl-2,2,6,6-tetramethyl piperidin-4-yl)adipat, bis(1-Oxyl-2,2,6,6-tetramethylpiperidin-4-yl)sebacat, bis(1-Oxyl-2,2,6,6-tetramethylpiperidin-4-yl)n-butylmalonat, bis(1-Oxyl-2,2,6,6-tetramethylpiperidin-4-yl)phthalat, bis(1-Oxyl-2,2,6,6-tetramethylpiperidin-4-yl)isophthalat, bis(1-Oxyl-2,2,6,6-tetramethylpiperidin-4-yl)terephthalat, bis(1-Oxyl-2,2,6,6-tetramethylpiperidin-4-yl)hexahydroterephthalat, N,N'-bis(1-Oxyl-2,2,6,6-tetramethylpiperidin-4-yl)adipamid, N-(1-Oxyl-2,2,6,6-tetramethylpiperidin-4-yl)-caprolactam, N-(1-Oxyl-2,2,6,6-tetramethylpiperidin-4-yl)-dodecylsuccinimid, 2,4,6-tris(1-Oxyl-2,2,6,6-tetramethylpiperidin-4-yl)isocyanurat, 2,4,6-tris-[N-butyl-N-1-oxyl-2,2,6,6-tetramethylpiperidin-4-yl]-s-triazin, 4,4'-Ethylenbis(1-oxyl-2,2,6,6-tetramethylpiperazin-3-on) und Kombinationen daraus.

4. Verfahren nach Anspruch 3, wobei das Nitroxylradikal-basierte Stabilisierungsmittel ausgewählt ist aus der Gruppe, bestehend aus 2,2,6,6-Tetramethyl-1-piperidinyloxy (TEMPO),4-Hydroxy-2,2,6,6-tetramethyl-1-piperidinyloxy (4H-TEMPO) und Kombinationen daraus.

5. Verfahren nach einem der vorhergehenden Ansprüche, wobei die Porogenzusammensetzung weniger als 1 ppm Benzen umfasst

## Revendications

1. Procédé de préparation d'une composition porogène comprenant pour 100 % en poids :
- au moins 99,5 % en poids de norbornadiène ;
- de 10 ppm à 500 ppm en poids d'un stabilisant à base d'un radical nitroxyle ;
- de 250 ppb à 10 ppm en poids de benzène,
ledit procédé comprenant :
l'étape a) de fourniture d'une solution de norbornadiène contenant du benzène comprenant plus de 100 ppm de benzène,
l'étape b) de séparation du benzène du norbornadiène par distillation extractive,
l'étape c) d'addition à la solution de norbornadiène issue de l'étape b) d'un stabilisant à base d'un radical nitroxyle.

2. Procédé selon la revendication 1, dans lequel la composition porogène comprend au moins 99,95 % de norbornadiène.

3. Procédé selon l'une des revendications précédentes, dans lequel le stabilisant à base d'un radical nitroxyle est choisi dans le groupe constitué de 2,2,6,6-tétraméthyl-1-pipéridinyloxy (TEMPO), 4-hydroxy-2,2,6,6-tétraméthyl-1-pipéridinyloxy (4H-TEMPO), di-tert-butyl-nitroxyle, 1-oxyl-2,2,6,6-tétraméthylpipéridin-4-one, acétate de 1-oxyl-2,2,6,6-tétraméthyl-pipéridin-4-yle, 2-éthylhexanoate de 1-oxyl-2,2,6,6-tétraméthylpipéridin-4-yle, stéarate de 1-oxyl-2,2,6,6-tétraméthylpipéridin-4-yle, benzoate de 1-oxyl-2,2,6,6-tétraméthylpipéridin-4-yle, 4-tert-butylbenzoate de 1-oxyl-2,2,6,6-tétraméthylpipéridin-4-yle, bis(1-oxyl-2,2,6,6-tétraméthylpipéridin-4-yl)-succinate, bis(1-oxyl-2,2,6,6-tétraméthylpipéridin-4-yl)adipate, bis(1-oxyl-2,2,6,6-tétraméthylpipéridin-4-yl)sébacate, bis(1-oxyl-2,2,6,6-tétraméthylpipéridin-4-yl)n-butylmalonate, bis(1-oxyl-2,2,6,6-tétraméthyl-pipéridin-4-yl)phtalate, bis(1-oxyl-2,2,6,6-tétraméthylpipéridin-4-yl)isophtalate, bis(1-oxyl-2,2,6,6-tétraméthylpipéridin-4-yl)téréphtalate, bis(1-oxyl-2,2,6,6-tétraméthylpipéridin-4-yl)hexahydrotéréphtalate, N,N'-bis(1-oxyl-2,2,6,6-tétraméthylpipéridin-4-yl)adipamide, N-(1-oxyl-2,2,6,6-tétraméthylpipéridin-4-yl)-caprolactame, N-(1-oxyl-2,2,6,6-tétraméthylpipéridin-4-yl)-dodécylsuccinimide, isocyanurate de 2,4,6-tris(1-oxyl-2,2,6,6-tétraméthylpipéridin-4-yl), 2,4,6-tris-[N-butyl-N-1-oxyl-2,2,6,6-tétraméthylpipéridin-4-yl]-s-triazine, 4,4'-éthylène-bis(1-oxyl-2,2,6,6-tétraméthylpipérazin-3-one) et leurs combinaisons.

4. Procédé selon la revendication 3, dans lequel le stabilisant à base d'un radical nitroxyle est choisi dans le groupe constitué de 2,2,6,6-tétraméthyl-1-pipéridinyloxy (TEMPO), 4-hydroxy-2,2,6,6-tétraméthyl-1-pipéridinyloxy (4H-TEMPO) et leurs combinaisons.

5. Procédé selon l'une quelconque des revendications précédentes, dans lequel la composition porogène comprend moins de 1 ppm de benzène.
